# EUROPÄISCHE PATENTSCHRIFT

(11) **EP 2 799 045 B1**
(45) Veröffentlichungstag und Bekanntmachung des Hinweises auf die Patenterteilung: **23.01.2019**
(21) Anmeldenummer: 13002267.6
(22) Anmeldetag: 29.04.2013
(51) Int. Cl.: A61F 9/008

(54) **Vorrichtung zur Bearbeitung von Augengewebe mittels Laserpulsen**
Device for processing eye tissue using laser pulses
Dispositif de traitement de tissus oculaires au moyen d'impulsions laser

(43) Veröffentlichungstag der Anmeldung: 05.11.2014
(73) Patentinhaber: Ziemer Ophthalmic Systems AG, 2562 Port (CH)
(72) Erfinder: Rathjen, Christian, 28197 Bremen (DE)
(74) Vertreter: Rentsch Partner AG

(56) Entgegenhaltungen:
- WO-A2-2007/064858
- WO-A2-2012/135579

## Beschreibung

### Technisches Gebiet

Die vorliegende Erfindung betrifft eine ophthalmologische Vorrichtung zur Bearbeitung von Augengewebe mittels Laserpulsen. Die vorliegende Erfindung betrifft insbesondere eine ophthalmologische Vorrichtung zur Bearbeitung von Augengewebe mittels Laserpulsen mit einer Projektionsoptik zur fokussierten Projektion der Laserpulse ins Augengewebe und einer der Projektionsoptik nachgeschalteten Scannvorrichtung zum Ablenken der von der Projektionsoptik projizierten Laserpulse in mindestens eine Ablenkrichtung.

### Stand der Technik

Ophthalmologische Vorrichtungen zur Bearbeitung von Augengewebe mittels Laserpulsen, bei denen die Scannvorrichtung der Projektionsoptik nachgeschaltet ist, haben den Vorteil einer einfachen Fokussieroptik. Sie weisen aber den Nachteil auf, dass sich durch die Nachschaltung der Scannvorrichtung eine Bildfeldwölbung, also eine gekrümmte Bearbeitungsfläche ergibt. Zur Kompensation dieser Bildfeldwölbung muss der Fokus der von der Scannvorrichtung abgelenkten Laserpulse korrigiert werden.

Die US 2011 /245814 beschreibt eine Vorrichtung, welche eine nachgeschaltete Scannvorrichtung mit einem einzigen kardanisch aufgehängten Spiegel aufweist, was kurze Arbeitsabstände und eine starke Fokussierung ermöglicht, wie sie z.B. in der Ophthalmologie zur Linsenchirurgie von Interesse sind. Nachteilig an dieser Anordnung ist allerdings die begrenzte Dynamik durch sich mitdrehende Antriebe. Überdies liegt die Spiegeloberfläche gemäss US 2011/245814 nicht im Drehzentrum, was zu einer zusätzlichen Verzerrung der Bildfeldwölbung führt, weil sich der Spiegel beim Scannen entlang der optischen Achse verschiebt.

WO 2007/064858 beschreibt ein ophthalmologisches Behandlungssystem mit einem Scanner und einem nachgeschalteten Spiegel. Der Scanner umfasst zwei optische Scann-Elemente zum Ablenken des Strahls, z.B. bewegliche Spiegel, Keile und/oder Linsen, die sich drehen oder in senkrechten X, Y Richtungen bewegen. Gemäss WO 2006/064858 fokussiert eine Linse den vom Scanner abgelenkten Strahl auf den nachgeschalteten Spiegel, welcher gekippt oder (wenn er eine Oberflächenkrümmung aufweist) verschoben wird, um als Scanner zu dienen oder die bereits durch den vorgeschalteten Scanner erzeugte Strahlbewegung zu verbessern. Vom Spiegel wird der Strahl über eine ophthalmologische Linse auf das Zielgewebe gelenkt.

### Darstellung der Erfindung

Es ist eine Aufgabe der vorliegenden Erfindung eine ophthalmologische Vorrichtung zur Bearbeitung von Augengewebe mittels Laserpulsen vorzuschlagen, welche eine Projektionsoptik mit nachgeschalteter Scannvorrichtung hat und zumindest einige Nachteile der bekannten Systeme nicht aufweist.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

Die ophthalmologische Vorrichtung zur Bearbeitung von Augengewebe mittels Laserpulsen umfasst eine Projektionsoptik zur fokussierten Projektion der Laserpulse und eine der Projektionsoptik nachgeschaltete Scannvorrichtung mit einem beweglichen Spiegel zum Ablenken der von der Projektionsoptik projizierten Laserpulse in mindestens eine Ablenkrichtung.

Die oben genannten Ziele werden durch die vorliegende Erfindung insbesondere dadurch erreicht, dass die ophthalmologische Vorrichtung überdies ein Antriebssystem umfasst, das eingerichtet ist, den Spiegel parallel zu verschieben.

In einer Ausführungsvariante umfasst das Antriebssystem einen mit der Scannvorrichtung gekoppelten Antrieb, der eingerichtet ist, die Scannvorrichtung entlang der optischen Achse der Projektionsoptik oder transversal zur optischen Achse der Projektionsoptik zu verschieben.

In einer Ausführungsvariante umfasst das Antriebssystem mindestens einen mit dem Spiegel gekoppelten Antrieb, der eingerichtet ist, den Spiegel parallel zu verschieben.

In einer Ausführungsvariante weist der Spiegel einen auf der optischen Achse der Projektionsoptik liegenden Drehpunkt auf, und das Antriebssystem ist eingerichtet, den Spiegel und den Drehpunkt entlang der optischen Achse der Projektionsoptik zu verschieben.

In einer Ausführungsvariante umfasst das Antriebssystem mehrere mit dem Spiegel gekoppelte Linearantriebe, welche eingerichtet sind, den Spiegel parallel zu verschieben, und den Spiegel um mindestens einen Drehpunkt zu drehen.

In einer Ausführungsvariante umfasst das Antriebssystem mehrere mit dem Spiegel gekoppelte Linearantriebe, welche eingerichtet sind, den Spiegel parallel zu verschieben, und den Spiegel um einen auf der optischen Achse der Projektionsoptik liegenden Drehpunkt zu drehen.

In einer Ausführungsvariante umfasst die ophthalmologische Vorrichtung ein Steuermodul, das eingerichtet ist, das Antriebssystem derart zu steuern, dass das Antriebssystem den Spiegel parallel an einen Schnittpunkt mit der optischen Achse der Projektionsoptik verschiebt, welcher eine definierte Distanz zur Brennweite der Projektionsoptik aufweist.

In einer Ausführungsvariante umfasst die ophthalmologische Vorrichtung ein Steuermodul, das eingerichtet ist, das Antriebssystem derart zu steuern, dass das Antriebssystem den Spiegel parallel an einen Schnittpunkt mit der optischen Achse der Projektionsoptik verschiebt, welcher eine definierte Distanz zur Brennweite der Projektionsoptik aufweist, und die Scannvorrichtung derart zu steuern, dass die Scannvorrichtung den Spiegel zur Ablenkung der Laserpulse in Richtung eines die definierte Distanz zum Schnittpunkt aufweisenden Zielpunkts im Augengewebe ausrichtet.

In einer Ausführungsvariante umfasst die ophthalmologische Vorrichtung ein Steuermodul, das eingerichtet ist, die Linearantriebe derart zu steuern, dass die Linearantriebe den Spiegel parallel an einen Schnittpunkt mit der optischen Achse der Projektionsoptik verschieben, welcher eine definierte Distanz zur Brennweite der Projektionsoptik aufweist, und den Spiegel so um den Schnittpunkt dreht, dass die Laserpulse in Richtung eines die definierte Distanz zum Schnittpunkt aufweisenden Zielpunkts im Augengewebe abgelenkt werden. Dabei ist losgelöst von der betreffenden Ausführungsvariante, allgemein anzumerken, dass zur fokussierten Projektion eines Laserpulses auf einen betreffenden Zielpunkt die Verschiebung und die Drehung des Spiegels nicht als sequentielle Schritte ausgeführt werden müssen, sondern durchaus parallel oder in umgekehrter Sequenz durchgeführt werden können.

In einer Ausführungsvariante ist das Steuermodul eingerichtet, das Antriebssystem und die Scannvorrichtung derart zu steuern, dass die Laserpulse auf Zielpunkte einer dreidimensionalen Bearbeitungsfläche im Augengewebe abgelenkt werden.

In einer Ausführungsvariante umfasst die ophthalmologische ein Korrektursystem, das eingerichtet ist, mittels eines der Projektionsoptik vorgeschalteten optischen Elements, eine von der Ablenkung der Laserpulse abhängige Brennweitenänderung vorzunehmen.

In einer Ausführungsvariante umfasst das Korrektursystem einen der Projektionsoptik vorgeschalteten Divergenzmodulator, welcher eingerichtet ist, die Divergenz des Laserstrahls abhängig von der Ablenkung der Laserpulse zu verändern. Durch die Veränderung der Divergenz des Laserstrahls ermöglicht der Divergenzmodulator eine Verschiebung des Fokus (entlang der Projektionsrichtung).

In einer Ausführungsvariante umfasst der Divergenzmodulator zwei seriell angeordnete optische Linsen, wobei mindestens eine der Linsen zur Modulation der Divergenz des Laserstrahls, auf einer optischen Achse verschiebbar, mit einem Bewegungstreiber gekoppelt ist; eine verformbare Linse; ein verformbares Spiegelelement; einen räumlichen Lichtmodulator zum Modulieren der Wellenfront des Laserstrahls; einen Flächenlichtmodulator zum Modulieren der Reflexionswinkel an mehreren Punkten einer Reflexionsfläche; einen Brechungsmodulator zum Modulieren des Brechungsindexes eines optischen Elements an mehreren Punkten im Querschnitt des Strahlengangs; und einen Amplitudenmodulator zur Amplitudenmodulation an mehreren Punkten im Querschnitt des Strahlengangs des Laserstrahls.

In einer Ausführungsvariante umfasst die ophthalmologische Vorrichtung ein der Projektionsoptik vorgeschaltetes oder in die Projektionsoptik integriertes Zoomsystem, das eingerichtet ist, eine von der Ablenkung der Laserpulse abhängige Brennweitenänderung vorzunehmen.

In einer Ausführungsvariante umfasst die ophthalmologische Vorrichtung ein Kompensationssystem, das bewegbare Massen zur Kompensation von durch bewegte optische Elemente verursachten Beschleunigungskräften umfasst.

### Kurze Beschreibung der Zeichnungen

Nachfolgend werden Ausführungen der vorliegenden Erfindung anhand von Beispielen beschrieben. Die Beispiele der Ausführung werden durch die folgenden beigelegten Figuren illustriert:
- Figur 1:: zeigt schematisch im Querschnitt eine ophthalmologische Vorrichtung zur Bearbeitung von Augengewebe mittels Laserpulsen, welche eine der Projektionsoptik nachgeschaltete Scannvorrichtung umfasst.
- Figur 2:: zeigt schematisch im Querschnitt eine ophthalmologische Vorrichtung zur Bearbeitung von Augengewebe mittels Laserpulsen, welche eine der Projektionsoptik nachgeschaltete Scannvorrichtung und ein der Scannvorrichtung nachgeschaltetes optisches Korrekturelement umfasst.
- Figur 3:: zeigt schematisch im Querschnitt eine ophthalmologische Vorrichtung zur Bearbeitung von Augengewebe mittels Laserpulsen, welche eine der Projektionsoptik nachgeschaltete Scannvorrichtung und ein der Scannvorrichtung nachgeschaltetes, in eine Patientenschnittstelle integriertes optisches Korrekturelement umfasst.
- Figur 4:: zeigt schematisch im Querschnitt eine ophthalmologische Vorrichtung zur Bearbeitung von Augengewebe mittels Laserpulsen, welche eine der Projektionsoptik nachgeschaltete Scannvorrichtung und ein Antriebssystem zum Bewegen der Scannvorrichtung umfasst.
- Figuren 5:: zeigt schematisch im Querschnitt eine ophthalmologische Vorrichtung zur Bearbeitung von Augengewebe mittels Laserpulsen, welche eine der Projektionsoptik nachgeschaltete Scannvorrichtung und ein Antriebssystem zum parallelen Verschieben eines Spiegels der Scannvorrichtung umfasst, welcher um einen von der optischen Achse der Projektionsoptik entfernt angeordneten Drehpunkt drehbar ist.
- Figur 6:: zeigt schematisch im Querschnitt eine ophthalmologische Vorrichtung zur Bearbeitung von Augengewebe mittels Laserpulsen, welche eine der Projektionsoptik nachgeschaltete Scannvorrichtung und ein Antriebssystem zum parallelen Verschieben eines Spiegels der Scannvorrichtung umfasst, welcher um einen auf der optischen Achse der Projektionsoptik liegenden Drehpunkt drehbar ist.
- Figur 7:: illustriert schematisch das Ablenken eines Laserpulses auf einen Zielpunkt im Augengewebe durch Drehen des Spiegels der der Projektionsoptik nachgeschalteten Scannvorrichtung.
- Figur 8:: illustriert schematisch das Abtasten von Zielpunkten auf einer Bearbeitungsfläche im Augengewebe durch paralleles Verschieben und. Drehen des Spiegels der der Projektionsoptik nachgeschalteten Scannvorrichtung.
- Figur 9:: zeigt schematisch im Querschnitt eine ophthalmologische Vorrichtung zur Bearbeitung von Augengewebe mittels Laserpulsen, welche eine der Projektionsoptik nachgeschaltete Scannvorrichtung und ein Korrektursystem zur von der Ablenkung der Laserpulse abhängigen Brennweitenänderung umfasst.
- Figur 10:: zeigt schematisch im Querschnitt eine ophthalmologische Vorrichtung zur Bearbeitung von Augengewebe mittels Laserpulsen, welche eine der Projektionsoptik nachgeschaltete Scannvorrichtung und ein Zoomsystem zur von der Ablenkung der Laserpulse abhängigen Brennweitenänderung umfasst.

### Wege zur Ausführung der Erfindung

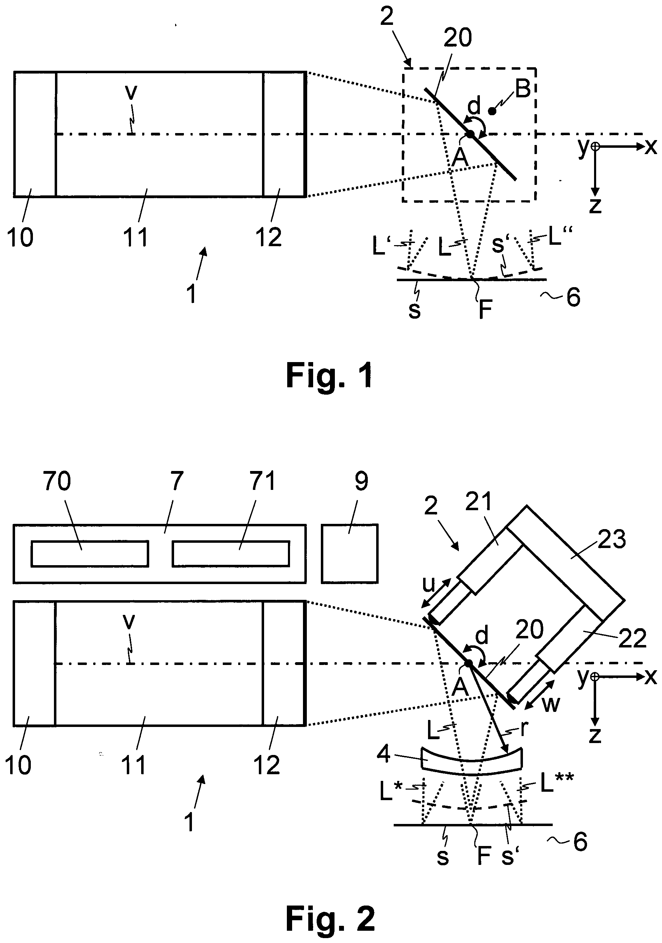

In den Figuren 1-10 bezieht sich das Bezugszeichen 1 jeweils auf eine ophthalmologische Vorrichtung zur Bearbeitung von Augengewebe 6 mittels Laserpulsen.

Die ophthalmologische Vorrichtung 1 umfasst eine Laserquelle 10 zur Erzeugung der Laserpulse, vorzugsweise Femtosekundenlaserpulse, zur Bearbeitung von Augengewebe 6, sowie eine Projektionsoptik 12 zur fokussierten Projektion der Laserpulse. Die Laserpulse werden von der Laserquelle 10 über ein optisches Übertragungssystem 11 der Projektionsoptik 12 zugeführt.

Wie in den Figuren 1-10 schematisch dargestellt ist, umfasst die ophthalmologische Vorrichtung 1 eine Scannvorrichtung 2, die der Projektionsoptik 12 nachgeschaltet ist. Die Scannvorrichtung 2 umfasst mindestens einen beweglichen Spiegel 20, zum Ablenken der von der Projektionsoptik 12 projizierten Laserpulse in mindestens eine Ablenkrichtung durch Drehung d des Spiegels 20 um mindestens eine Drehachse. Die Scannvorrichtung 2 ist vorzugsweise eingerichtet, den Spiegel 20 um mehrere Drehachsen zu bewegen, die durch einen Drehpunkt A verlaufen, welcher auf der optischen Achse v der Projektionsoptik 12 liegt. Wie später beschrieben wird, ist der Drehpunkt A auf der optischen Achse v verschiebbar. In alternativen Ausführungsvarianten ist der Spiegel 20 um eine oder mehrere Drehachsen drehbar, die durch einen ausserhalb der optischen Achse v liegenden Drehpunkt B verlaufen.

Wie in den Figuren 2, 3, 5, 6 schematisch dargestellt ist, umfasst die Scannvorrichtung 2 in einer Variante ein Antriebssystem mit mehreren Linearantrieben 21, 22, die mit dem Spiegel 20 gekoppelt sind. Die Antriebe sind beispielsweise als piezo-elektrische oder elektromagnetische Antriebe ausgeführt. Die ophthalmologische Vorrichtung umfasst ein Steuermodul 23, das eingerichtet ist, die Linearantriebe 21, 22 so zu steuern, dass sie den Spiegel 20 über entsprechend gegenläufige Translationsbewegungen u, w um einen vorzugsweise auf der optischen Achse v der Projektionsoptik 12 und der spiegelnden Oberfläche des Spiegels 20 (Spiegeloberfläche) liegenden Drehpunkt A drehen. Mehr als zwei Linearantriebe 21, 22 ermöglichen Drehungen d um verschiedene durch den Drehpunkt A verlaufende Achsen im x/y/z-Raum.

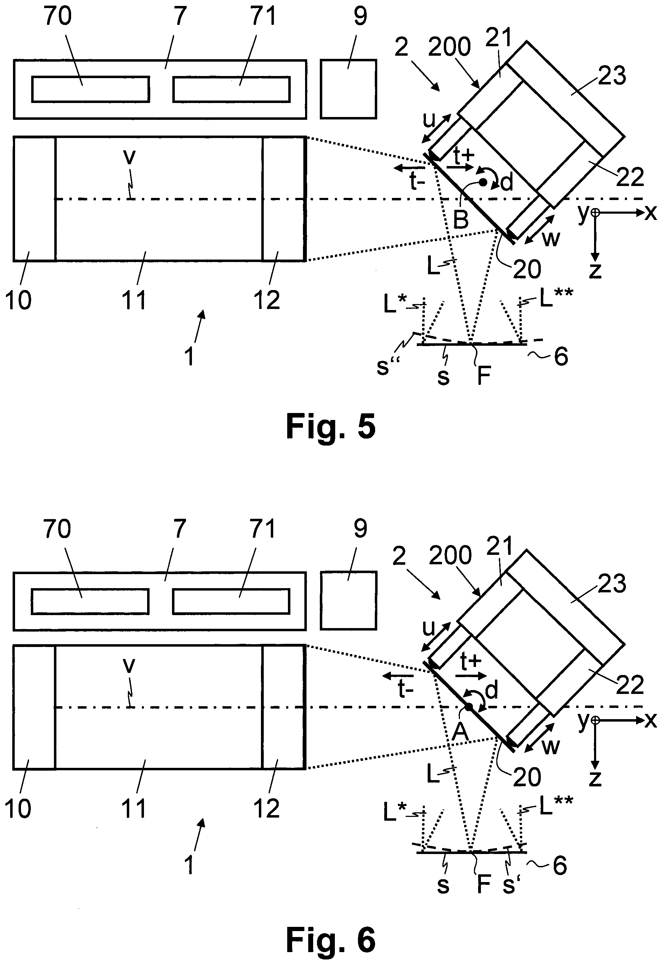

Wie in der Figur 1 illustriert ist, bewirkt die Drehung d des Spiegels 20 der nachgeschalteten Scannvorrichtung 2 eine Wölbung des Bildfelds respektive einer Sollbearbeitungsfläche s. Mit anderen Worten, der Fokus F eines durch die Projektionsoptik 12 fokussierter Laserstrahls L bewegt sich bei einer Drehung d des Spiegels 20 um eine senkrecht zur Zeichenebene verlaufende Drehachse auf einem Kreisbogen s' respektive bei einer Drehung d des Spiegels um mehrere, durch einen auf der optischen Achse v liegenden Drehpunkt A verlaufende, Drehachsen auf einer Kugelschale, wie in der Figur 1 mit den abgelenkten Laserstrahlen L', L" angedeutet wird, wobei der Kreisbogen s' respektive die Kugelschale dem gewölbten respektive verzerrten Bildfeld entsprechen. Bei einer Drehung um einen ausserhalb der optischen Achse v angeordneten Drehpunkt B, wird eine zusätzliche Verzerrung der Bildfeldwölbung bewirkt, wie beispielsweise in der Figur 5 mit dem Kreisbogen s" dargestellt ist, da der Spiegel 20 durch die Drehbewegung überdies entlang der optischen Achse v verschoben wird.

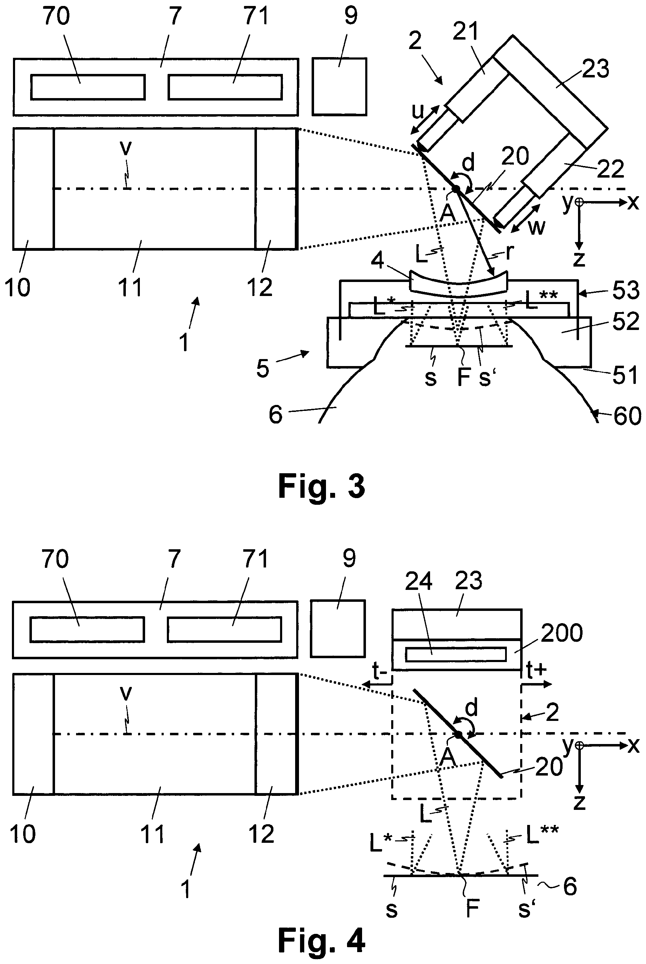

In den in den Figuren 2 und 3 illustrierten Ausführungsvarianten umfasst die ophthalmologische Vorrichtung 1 ein der Scannvorrichtung 2 nachgeschaltetes optisches Korrekturelement 4, das eingerichtet ist, die von der Scannvorrichtung 2 abgelenkten Laserpulse fokussiert auf eine Sollbearbeitungsfläche s im Augengewebe 6 abzubilden, beispielsweise auf eine Sollbearbeitungsebene zur Erzeugung eines ebenen Schnitts, wie in der Figur 2 durch die korrigierten Laserstrahlen L*, L** angedeutet ist. In einer Variante ist das Korrekturelement 4 als optisches Linsenelement ausgestaltet. Das Linsenelement weist beispielsweise eine zum Drehpunkt A des Spiegels 20 äquidistante Linsenoberfläche auf, wie in der Figur 2 mit dem Radius r angedeutet ist.

Zur Vergrösserung des Abstands der Scannvorrichtung 2 vom Auge des Patienten ist das optische Korrekturelement 4 in einer Ausführungsvariante als Linsenelement ausgeführt, das so ausgestaltet ist, dass die von der Scannvorrichtung 2 abgelenkten Laserpulse fokussiert auf eine ausserhalb der Brennweite f der Projektionsoptik 12 liegenden Sollbearbeitungsfläche abgebildet werden. Das heisst das Linsenelement des optischen Korrekturelements 4 bewirkt eine definierte Verschiebung respektive Vergrösserung der Brennweite f der Projektionsoptik 12. In einer weiteren Variante ist das Korrekturelement 4 zusätzlich eingerichtet, die Brechkraft zu erhöhen und damit den Laserstrahl verstärkt zu fokussieren, d.h. auf einen Fokus mit reduzierter Spotgrösse zu projizieren. Die Projektionsoptik 12 und das optische Korrekturelement 4 sind in einer Variante gezielt aufeinander abgestimmt, um als kombinierte Projektionsoptik eine definierte Spotqualität des projizierten Laserpulses respektive Laserstrahls zu erreichen, beispielsweise hinsichtlich Grösse und Form (Durchmesser quer zur Projektionsrichtung, Länge in Projektionsrichtung).

Für Konfigurationen und/oder Anwendungen, bei denen der Spiegel 20 um einen ausserhalb der optischen Achse v der Projektionsoptik 12 liegenden Drehpunkt B gedreht wird, wird ein als anamorphotisches Element ausgestaltetes Korrekturelement 4 eingesetzt, da sich bei Drehungen des Ablenkspiegels der Scannvorrichtung 2 um einen ausserhalb der optischen Achse v der Projektionsoptik 12 liegenden Drehpunkt B weitere Verzerrungen des Bildfelds ergeben, die durch eine Asymmetrie bezüglich der Projektionsachse charakterisiert sind (siehe beispielsweise in der Figur 5 Kreisbogen s" um Drehpunkt B).

In verschiedenen Ausführungsvarianten ist das optische Korrekturelement 4 fest oder auswechselbar mit der ophthalmologischen Vorrichtung 1 respektive mit einer Patientenschnittstellenvorrichtung 5 der ophthalmologischen Vorrichtung 1 verbunden. Es ist somit möglich, auswechselbar unterschiedliche Korrekturelemente 4 in die Patientenschnittstellenvorrichtung 5 einzusetzen und für die Bearbeitung des Augengewebes 6 zu verwenden.

Wie in der Figur 3 schematisch illustriert wird, umfasst die Patientenschnittstellenvorrichtung 5 beispielsweise einen Vakuumapplikator 51, z.B. einen Saugring, zur Befestigung am Auge 60 des Patienten. In verschiedenen Ausführungsvarianten umfasst die Patientenschnittstellenvorrichtung 5 einen Kontaktkörper 52, beispielsweise einen Applanationskörper, zur Kontaktierung auf der Hornhaut des Auges 60, oder einen mit Flüssigkeit füllbaren Hohlraum, der eine Öffnung aufweist, welche im auf dem Auge 60 angebrachten Zustand der Patientenschnittstellenvorrichtung 5 durch das Auge 60 respektive die Hornhaut abgeschlossen wird. In einer Variante ist das optische Korrekturelement 4 als Kontaktkörper ausgeführt, je nach Variante und/oder Anwendung als Applanationskörper mit flacher Kontaktfläche oder als Kontaktkörper mit gekrümmter Kontaktfläche. In einer weiteren Ausführungsvariante bilden das optische Korrekturelement 4 und ein separater Applanationskörper ein optisches Korrektursystem, das eingerichtet ist, die von der Scannvorrichtung 2 abgelenkten Laserpulse fokussiert auf die Sollbearbeitungsfläche s im Augengewebe 6 abzubilden.

Die Patientenschnittstellenvorrichtung 5 ist in einer Variante mit der ophthalmologischen Vorrichtung 1 verbunden. Zur ergonomischen Verbesserung der Applizierbarkeit am Auge 60 ist die Patientenschnittstellenvorrichtung 5 drehbar gelagert, beispielsweise um zwei Drehachsen. In einer Ausführungsvariante ist die Patientenschnittstellenvorrichtung 5 um den Drehpunkt A des Spiegels 20 drehbar gelagert, das heisst die Drehachsen der Drehgelenke der Patientenschnittstellenvorrichtung 5 verlaufen durch den Drehpunkt A des Spiegels 20. Eine der Drehachsen der Drehgelenke der Patientenschnittstellenvorrichtung 5 entspricht beispielsweise der optischen Achse v der Projektionsoptik 12 respektive eines später beschriebenen Zoomsystems 83, so dass die Patientenschnittstellenvorrichtung 5 um die optische Achse v (und bei auf der optischen Achse v liegendem Drehpunkt A des Spiegels 20 auch um diesen Drehpunkt A) drehbar ist.

In einer Ausführungsvariante ist die Scannvorrichtung 2 so ausgeführt und mit der ophthalmologischen Vorrichtung 1 verbunden, dass sie über eine Verstellvorrichtung aus dem Strahlengang wegbewegbar ist, beispielsweise über eine Drehung um ein Drehgelenk oder eine Translation, so dass das Auge 60 einsehbar ist. Dazu umfasst die Verstellvorrichtung ein an der ophthalmologischen Vorrichtung 1 angebrachtes Drehlager respektive eine Schiene, mittels derer die Scannvorrichtung 2 aus dem Strahlengang wegbewegbar und wieder in den Strahlengang einkoppelbar ist, manuell oder mittels eines Antriebsystems, so dass im in den Strahlengang eingekoppelten Zustand der Scannvorrichtung 2 die von der Projektionsoptik 12 zugeführten Laserpulse wieder präzise vom Spiegel 20 der Scannvorrichtung 2 auf Zielpunkte F ablenkbar sind. Dabei soll festgehalten werden, dass im Zustand, wo die Scannvorrichtung 2 aus dem Strahlengang wegbewegt ist, eine ungehinderte Sicht auf das Auge 60 möglich ist, sowohl im am Auge 60 befestigten Zustand der Patientenschnittstellenvorrichtung 5 als auch wenn die Patientenschnittstellenvorrichtung 5 nicht am Auge 60 befestigt oder das Auge 60 kontaktiert, sondern bloss über dem Auge 60 angeordnet ist.

In einer weiteren Ausführungsvariante umfasst die ophthalmologische Vorrichtung 1 ein Detektionsmodul 9, welches eingerichtet ist, das optische Korrekturelement 4 zu detektieren und ein Steuermodul umfasst, welches eingerichtet ist, die Einstellung der Scannvorrichtung 2, hinsichtlich der Lage respektive des Drehwinkels des Spiegels 20, abhängig von einer Detektierung des optischen Korrekturelements 4 zu steuern. Die Detektierung des optischen Korrekturelements 4 beinhaltet die Position des Korrekturelements 4 bezüglich der ophthalmologischen Vorrichtung 1 respektive der Scannvorrichtung 2, den Typ des Korrekturelements 4, optische Eigenschaften des Korrekturelements 4, welche beispielsweise dem Typ des Korrekturelements 4 zugeordnet sind, und/oder Abmessungen des Korrekturelements 4, insbesondere die Dicke des Korrekturelements 4. Zur Detektierung des optischen Korrekturelements 4 umfasst das Detektionsmodul 9, abhängig von der Ausführungsvariante und der zu detektierenden Parameter, einen oder mehrere optische Sensoren, Distanzsensoren, elektrische Sensoren, elektromechanische Sensoren und/oder elektromagnetische Sensoren, z.B. RFID-Sensoren. Das Steuermodul des Detektionsmoduls 9 ist insbesondere eingerichtet, die Scannvorrichtung 2 abhängig von der detektierten Position des Korrekturelements 4 zu steuern und dadurch die Lage respektive den Drehwinkel des Spiegels 20 dynamisch an Positionsänderungen des Korrekturelements 4 respektive des Patienten anzupassen um die Laserpulse abhängig von der Position respektive Positionsänderungen an definierte Zielpunkte F im Augengewebe 6 abzulenken. Das Steuermodul des Detektionsmoduls 9 ist überdies eingereichtet den Drehpunkt A des Spiegels 20 zur Patientenschnittstellenvorrichtung 5 auszurichten und die durch Drehgelenke der Patientenschnittstellenvorrichtung 5 bewirkte Verkippung der Patientenschnittstellenvorrichtung 5 zu kompensieren.

In den in den Figuren 4, 5 und 6 dargestellten Ausführungen weist die ophthalmologische Vorrichtung 1 ein Antriebssystem 200 auf, das eingerichtet ist, den Spiegel 20 parallel zu verschieben. Das Antriebssystem 200 ist insbesondere eingerichtet, den Spiegel 20 mit einer translatorischen Bewegungskomponente t-, t+ entlang der optischen Achse v zu verschieben. Durch die parallele Verschiebung des Spiegels 20 der der Projektionsoptik 12 nachgeschalteten Scannvorrichtung 2 wird die Distanz der Spiegeloberfläche zur Projektionsoptik 12 verändert, was bei unveränderter Brennweite f der Projektionsoptik 12 eine Verschiebung des Fokus in Projektionsrichtung bewirkt. Eine Verkleinerung der Distanz der Spiegeloberfläche zur Projektionsoptik 12 bewirkt eine Vergrösserung der Distanz des Fokus zur Spiegeloberfläche und ermöglicht somit eine fokussierte Projektion der Laserpulse auf tiefer liegende Zielpunkte F im Augengewebe 6 (Verschiebung in Projektions- respektive z-Richtung); eine Vergrösserung der Distanz der Spiegeloberfläche zur Projektionsoptik 12 hingegen bewirkt eine Verkürzung der Distanz des Fokus zur Spiegeloberfläche und ermöglicht somit eine fokussierte Projektion der Laserpulse auf höher liegende Zielpunkte F im Augengewebe 6 (Verschiebung entgegen der Projektions- respektive z-Richtung).

In der Ausführungsvariante nach Figur 4 umfasst das Antriebssystem 200 zum parallelen Verschieben des Spiegels 20 einen mit der Scannvorrichtung 2 gekoppelten Antrieb 24, der eingerichtet ist, die Scannvorrichtung 2 entlang der optischen Achse v der Projektionsoptik 12 oder quer zur optischen Achse v der Projektionsoptik 12 zu verschieben.

In der Ausführungsvariante der Figuren 5 und 6 umfasst das Antriebssystem 200 mindestens einen mit dem Spiegel 20 gekoppelten Antrieb 21, 22, der eingerichtet ist, den Spiegel 20 parallel zu verschieben. Das Antriebssystem 200 umfasst beispielsweise mehrere mit dem Spiegel 20 gekoppelte Linearantriebe 21, 22, welche eingerichtet sind, den Spiegel 20 parallel zu verschieben.

Wie in den Figuren 5 und 6 schematisch dargestellt ist, sind die Linearantriebe 21, 22 überdies eingerichtet, den Spiegel 20 mittels entsprechend gegenläufigen Translationsbewegungen u, w um mindestens einen Drehpunkt A, B zu drehen. In der Darstellung der Figur 5 erfolgt die Drehung d um einen ausserhalb der optischen Achse v der Projektionsoptik 12 liegenden Drehpunkt B. In der Darstellung der Figur 6 erfolgt die Drehung d um einen auf der optischen Achse v der Projektionsoptik 12 und der Spiegeloberfläche des Spiegels 20 liegenden Drehpunkt A. Mehr als zwei Linearantriebe 21, 22 ermöglichen Drehungen d um verschiedene durch den Drehpunkt A, B verlaufende Achsen im x/y/z-Raum. Das Antriebssystem 200 ermöglicht somit, den Spiegel 20 und seinen Drehpunkt A, B entlang der optischen Achse v der Projektionsoptik 12 zu verschieben und den Spiegel 20 um den Drehpunkt A, B zu drehen, um das Augengewebe 6 mittels fokussiert auf Zielpunkte F abgelenkter Laserpulse zu bearbeiten.

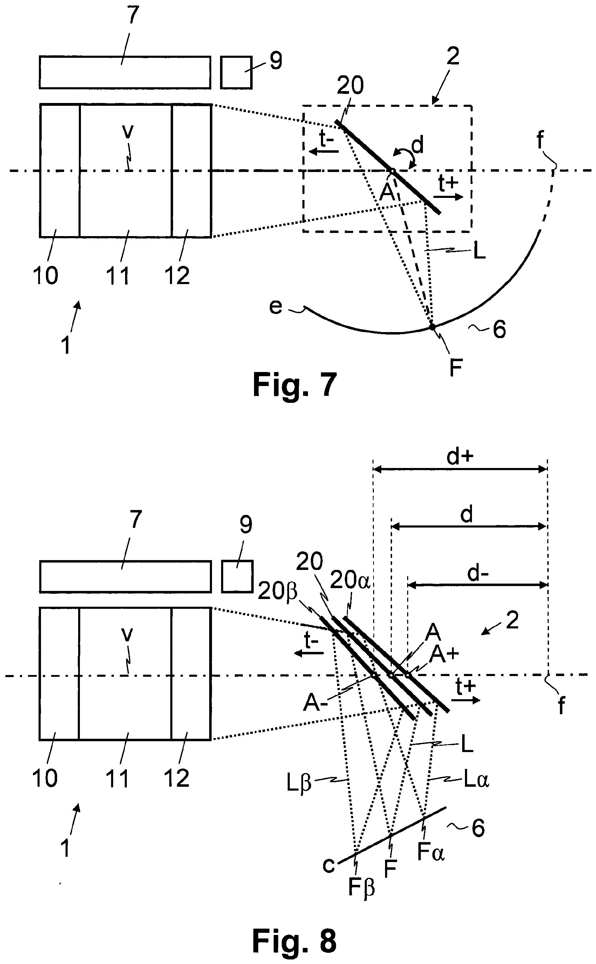

Die Figur 7 illustriert schematisch die Brennweite f der Projektionsoptik 12 auf der optischen Achse v und den sich dadurch definierten Kreisbogen e, auf den der abgelenkte Laserstrahl L bei einer Drehung d des Spiegels 20, um eine normal zur Zeichenebene durch den Drehpunkt A verlaufende Drehachse, fokussiert ablenkbar ist, respektive die durch die Brennweite definierte Kugelschale auf der der abgelenkte Laserstrahl L bei Drehungen d des Spiegels 20, um mehrere durch den Drehpunkt A verlaufende Drehachsen, fokussiert ablenkbar ist.

Die Figur 8 illustriert schematisch die Kombination einer Drehung d des Spiegels 20 um den auf der optischen Achse v und auf der Spiegeloberfläche liegenden Drehpunkt A und einer Bewegung des Spiegels 20 respektive des Drehpunkts A mit einer translatorischen Bewegungskomponente t-, t+ entlang der optischen Achse v. Die Figur 8 illustriert die Kombination von Drehung d und Verschiebung des Spiegels 20 am Beispiel der Bearbeitung von Zielpunkten Fα, F, Fß, die auf einer Bearbeitungslinie c im Augengewebe 6 liegen. Wie in der Figur 8 ersichtlich ist, steuert das Steuermodul 23 das Antriebssystem 200 respektive die Linearantriebe 21, 22 so, dass das Antriebssystem 200 zur Bearbeitung der Zielpunkte Fα, F, Fβ den Drehpunkt respektive den Spiegel 20 an einen Punkt A-, A, A+ auf der optischen Achse v der Projektionsoptik 12 verschiebt, der eine definierte Distanz d-, d, d+ zur Brennweite f der Projektionsoptik 12 aufweist. Der Drehpunkt A-, A, A+ liegt somit auf einem Schnittpunkt der Spiegeloberfläche mit der optischen Achse v, wobei die Distanz d-, d, d+ vom Drehpunkt A-, A, A+ respektive vom Schnittpunkt zur Brennweite f der Projektionsoptik 12 der Distanz des betreffenden Zielpunkts Fα, F, Fβ zum Drehpunkt A-, A, A+ respektive Schnittpunkt entspricht. Die Brennweite f der Projektionsoptik 12 und der betreffende Zielpunkt Fα, F, Fβ liegen somit jeweils auf einem gemeinsamen Kreisbogen e respektive auf einer gemeinsamen Kugelschale um den Drehpunkt A-, A, A+ respektive Schnittpunkt auf der optischen Achse v. Dabei wird der Spiegel 20 so um den Drehpunkt A-, A, A+ gedreht, dass der ausgelenkte Spiegel 20α, 20, 20ß den Laserstrahl Lα, L, Lβ in Richtung des Zielpunkts Fa, F, Fβ ablenkt. Somit wird der Laserstrahl Lα, L, Lβ respektive die Laserpulse von der Projektionsoptik 12 über den ausgelenkten Spiegel 20α, 20, 20β fokussiert auf den betreffenden Zielpunkt Fα, F, Fβ auf der Bearbeitungslinie c projiziert. Dabei soll hier angemerkt werden, dass die Kombination von Drehung d und Verschiebung nicht sequentiell sondern vorzugsweise parallel/gleichzeitig durchgeführt wird, so dass die Zielpunkte Fα, F, Fβ möglichst schnell anfokussiert und bearbeitet werden können.

Das Steuermodul 23 ist in einer Variante eingerichtet, das Antriebssystem 200 und die Scannvorrichtung 2 derart zu steuern, dass die Laserpulse fokussiert auf Zielpunkte Fα, F, Fβ einer dreidimensionalen Bearbeitungsfläche s im Augengewebe 6 abgelenkt und projiziert werden. Dadurch wird eine dreidimensionale Bearbeitung respektive Volumenbearbeitung erreicht.

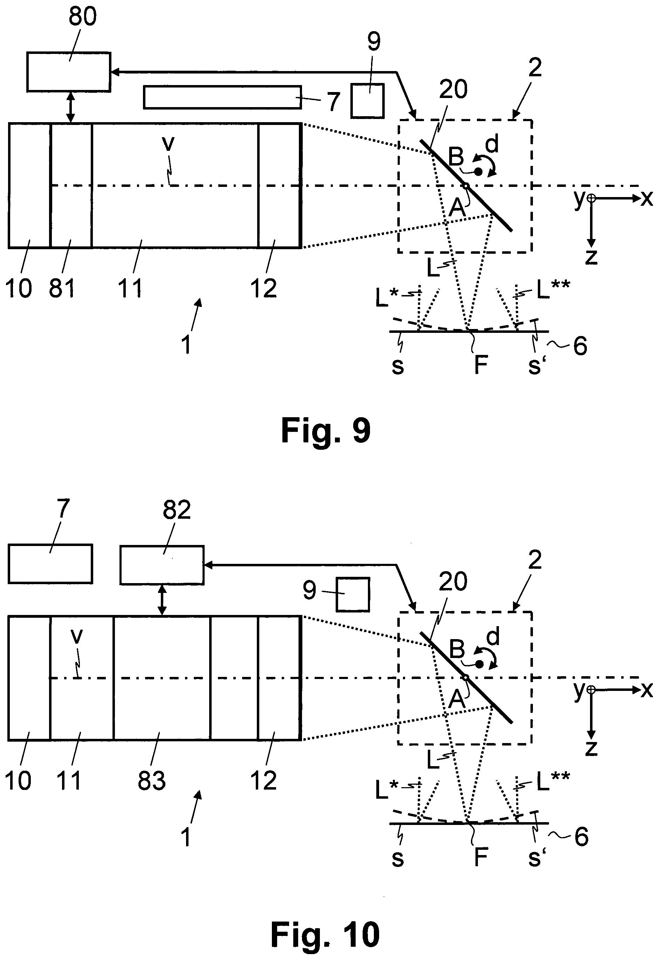

In den nachfolgenden Abschnitten werden mit Bezug zu den Figuren 9 und 10 Varianten der ophthalmologischen Vorrichtung 1 mit einem Korrektursystem beschrieben, das eingerichtet ist, mittels eines oder mehrerer der Projektionsoptik 12 vorgeschalteten oder in die Projektionsoptik 12 integrierter optischen Elemente, eine von der Ablenkung der Laserpulse abhängige Brennweitenänderung vorzunehmen.

Figur 9 illustriert eine Ausführungsvariante, in der die ophthalmologische Vorrichtung 1 als weiteres Merkmal im optischen Übertragungssystem 11 einen der Projektionsoptik 1 2 vorgeschalteten Divergenzmodulator 81 aufweist. Der Divergenzmodulator 81 ist eingerichtet, die Divergenz des Laserstrahls abhängig von der Ablenkung der Laserpulse so zu verschieben, dass von der Scannvorrichtung 2 verursachte Bildfeldverkrümmungen wenigstens teilweise kompensiert werden. Je nach Ausführungsvariante umfasst der Divergenzmodulator 81 zur Divergenzmodulation zwei seriell angeordnete optische Linsen, wobei mindestens eine der Linsen zur Modulation der Divergenz des Laserstrahls, auf der optischen Achse v verschiebbar, mit einem Bewegungstreiber gekoppelt ist; eine verformbare Linse; ein verformbares Spiegelelement; einen räumlichen Lichtmodulator zum Modulieren der Wellenfront des Laserstrahls; einen Flächenlichtmodulator zum Modulieren der Reflexionswinkel an mehreren Punkten einer Reflexionsfläche; einen Brechungsmodulator zum Modulieren des Brechungsindexes eines optischen Elements an mehreren Punkten im Querschnitt des Strahlengangs; und/oder einen Amplitudenmodulator zur Amplitudenmodulation an mehreren Punkten im Querschnitt des Strahlengangs des Laserstrahls. Der Divergenzmodulator 81 wird von einem Steuermodul 80 abhängig von der durch die Scannvorrichtung 2 ausgeführten Ablenkung der Laserpulse gesteuert, also abhängig von einem oder mehreren Auslenkungswinkeln von einem oder mehreren Spiegeln 20 der Scannvorrichtung 2. Dazu verwendet das Steuermodul 80 Korrekturparameter respektive Steuerwerte zur Steuerung des Divergenzmodulators 81, welche verschiedenen Auslenkungswinkeln zugeordnet gespeichert sind und eine entsprechende Divergenzmodulation zur Kompensation von Bildfeldverkrümmungen beim jeweils betreffenden Auslenkungswinkel bewirken, wie in der Figur 9 durch die korrigierten Laserstrahlen L*, L** angedeutet ist.

Figur 10 illustriert eine Ausführungsvariante, in der die ophthalmologische Vorrichtung 1 als weiteres Merkmal im optischen Übertragungssystem 11 ein der Projektionsoptik 12 vorgeschaltetes oder in die Projektionsoptik 12 integriertes Zoomsystem 83 aufweist. Das Zoomsystem 83 ist eingerichtet, eine von der Ablenkung der Laserpulse abhängige Brennweitenänderung vorzunehmen, um von der Scannvorrichtung 2 verursachte Bildfeldverkrümmungen wenigstens teilweise zu kompensieren. Das Zoomsystem 83 ist überdies eingerichtet, die Spotgrösse und/oder Aberrationen einzustellen. Das Zoomsystem 83 umfasst mindestens zwei individuell einstellbare Optiksysteme, beispielsweise zwei Linsengruppen mit jeweils einer oder mehreren bewegbaren Linsen, und/oder ein oder mehrere deformierbare Spiegel/Linsen und einschiebbare KorrekturElemente. Die Optiksysteme sind mit einem Antriebssystem gekoppelt, welches einen oder mehrere elektrische Motoren umfasst und eingerichtet ist, die Optiksysteme individuell einzustellen, beispielsweise durch Verschieben von Linsen entlang der optischen Achse v und/oder normal zur optischen Achse v (in den/aus dem Strahlengang). Das Zoomsystem 83 wird von einem Steuermodul 82 abhängig von der durch die Scannvorrichtung 2 ausgeführten Ablenkung der Laserpulse gesteuert, also abhängig von einem oder mehreren Auslenkungswinkeln von einem oder mehreren Spiegeln 20 der Scannvorrichtung 2. Dazu verwendet das Steuermodul 82 Korrekturparameter respektive Steuerwerte zur Steuerung des Zoomsystems 83, welche verschiedenen Auslenkungswinkeln zugeordnet gespeichert sind und eine entsprechende Brennweitenänderung zur Kompensation von Bildfeldverkrümmungen beim jeweils betreffenden Auslenkungswinkel bewirken, wie in der Figur 10 durch die korrigierten Laserstrahlen L*, L** angedeutet ist.

In verschiedenen Ausführungsvarianten ist die Projektionsoptik 12 als Zoomsystem 83 ausgestaltet oder das Zoomsystem 83 wird als Projektionsoptik 12 eingesetzt. In einer Variante wird die gesamte Projektionsoptik 12 abhängig von der Ablenkung der Laserpulse verschoben, um eine kompensierende Fokusverschiebung zu erwirken. In einer weiteren Ausführungsvariante sind ein Divergenzmodulator 81 und ein Zoomsystem 83 vorgesehen, welche so angesteuert werden, dass sie eine von der Ablenkung der Laserpulse abhängige Fokusverschiebung kombiniert durch Divergenzmodulation des Laserstrahls mittels des Divergenzmodulators 81 und Brennweitenänderung mittels des Zoomsystems 83 vornehmen.

In einer weiteren Ausführungsvariante umfasst die ophthalmologische Vorrichtung 1 ein Kompensationssystem 7, das bewegbare Massen 70 zur Kompensation von durch bewegte optische Elemente verursachten Beschleunigungskräften umfasst, um Vibrationen der ophthalmologischen Vorrichtung 1 möglichst zu vermeiden oder zumindest zu reduzieren. Das Kompensationssystem 7 umfasst einen oder mehrere mit den Massen 70 gekoppelte Antriebe 71, welche eingerichtet sind, die Massen 70 entsprechend der Steuerung durch ein Steuermodul gegenläufig zu den Bewegungen der optischen Elemente zu bewegen. Die Massen 70 werden beispielsweise zur Kompensation von Beschleunigungskräften eingerichtet, die durch die Bewegungen von optischen Elementen der Scannvorrichtung 2, z.B. Bewegungen des Spiegels 20 und/oder der Antriebe 21, 22, des Divergenzmodulators 81 und/oder des Zoomsystems 83 verursacht werden.

An dieser Stelle soll festgehalten werden, dass die in den obenstehenden Abschnitten angeführten und beschriebenen Steuermodule 23, 80, 82 jeweils einen Schaltkreis umfassen, beispielsweise einen (Mikro-)Prozessor, der durch Computercode eines auf einem (nicht-transienten) computerlesbaren Medium gespeicherten Programms gesteuert wird, oder eine andere programmierte Logikeinheit oder Steuerungselektronik. Die Steuermodule 23, 80, 82 generieren Steuersignale, beispielsweise abhängig von Steuerprogrammen und/oder Feedback-Signalen der Scannvorrichtung 2, des Divergenzmodulators 81 oder des Zoomsystems 83, zur Steuerung der Scannvorrichtung 2, des Antriebssystems 200, der Linearantriebe 21, 22, des Divergenzmodulators 81, des Zoomsystems 83 und/oder des Kompensationssystems 7.

## Patentansprüche

1. Ophthalmologische Vorrichtung (1) zur Bearbeitung von Augengewebe (6) mittels Laserpulsen, umfassend:
eine Projektionsoptik (12) zur fokussierten Projektion der Laserpulse ins Augengewebe, und
eine der Projektionsoptik (12) nachgeschaltete Scannvorrichtung (2) mit einem beweglichen Spiegel (20) zum Ablenken der von der Projektionsoptik (12) projizierten Laserpulse in mindestens eine Ablenkrichtung durch Drehung des Spiegels (20) um mindestens eine Drehachse,
**gekennzeichnet durch**
ein Antriebssystem (200), das eingerichtet ist, den Spiegel (20) parallel zu verschieben.

2. Ophthalmologische Vorrichtung (1) nach Anspruch 1, **dadurch gekennzeichnet, dass** das Antriebssystem (200) einen mit der Scannvorrichtung (2) gekoppelten Antrieb (24) umfasst, der eingerichtet ist, die Scannvorrichtung (2) entlang der optischen Achse (v) der Projektionsoptik (12) oder transversal zur optischen Achse (v) der Projektionsoptik (12) zu verschieben.

3. Ophthalmologische Vorrichtung (1) nach Anspruch 1, **dadurch gekennzeichnet, dass** das Antriebssystem (200) mindestens einen mit dem Spiegel (20) gekoppelten Antrieb (21, 22) umfasst, der eingerichtet ist, den Spiegel (20) parallel zu verschieben.

4. Ophthalmologische Vorrichtung (1) nach einem der Ansprüche 1 bis 3, **dadurch gekennzeichnet, dass** der Spiegel (20) einen auf der optischen Achse (v) der Projektionsoptik (12) liegenden Drehpunkt (A) aufweist, und dass das Antriebssystem (200) eingerichtet ist, den Spiegel (20) und den Drehpunkt (A) entlang der optischen Achse (v) der Projektionsoptik (12) zu verschieben.

5. Ophthalmologische Vorrichtung (1) nach Anspruch 1, **dadurch gekennzeichnet, dass** das Antriebssystem (200) mehrere mit dem Spiegel (20) gekoppelte Linearantriebe (21, 22) umfasst, welche eingerichtet sind, den Spiegel (20) parallel zu verschieben, und den Spiegel (20) um mindestens einen Drehpunkt (A, B) zu drehen.

6. Ophthalmologische Vorrichtung (1) nach Anspruch 1, **dadurch gekennzeichnet, dass** das Antriebssystem (200) mehrere mit dem Spiegel (20) gekoppelte Linearantriebe (21, 22) umfasst, welche eingerichtet sind, den Spiegel (20) parallel zu verschieben, und den Spiegel (20) um einen auf der optischen Achse (v) der Projektionsoptik (12) liegenden Drehpunkt (A) zu drehen.

7. Ophthalmologische Vorrichtung (1) nach einem der Ansprüche 1 bis 6, **gekennzeichnet durch** ein Steuermodul (23), das eingerichtet ist, das Antriebssystem (200) derart zu steuern, dass das Antriebssystem (200) den Spiegel (20) parallel an einen Schnittpunkt (A-, A, A+) mit der optischen Achse (v) der Projektionsoptik (12) verschiebt, welcher eine definierte Distanz (d-, d, d+) zur Brennweite (f) der Projektionsoptik (12) aufweist.

8. Ophthalmologische Vorrichtung (1) nach einem der Ansprüche 1 bis 6, **gekennzeichnet durch** ein Steuermodul (23), das eingerichtet ist, das Antriebssystem (200) derart zu steuern, dass das Antriebssystem (200) den Spiegel (20) parallel an einen Schnittpunkt (A-, A, A+) mit der optischen Achse (v) der Projektionsoptik (12) verschiebt, welcher eine definierte Distanz (d-, d, d+) zur Brennweite (f) der Projektionsoptik (12) aufweist, und die Scannvorrichtung (2) derart zu steuern, dass die Scannvorrichtung (2) den Spiegel (20) zur Ablenkung der Laserpulse in Richtung eines die definierte Distanz (d-, d, d+) zum Schnittpunkt (A-, A, A+) aufweisenden Zielpunkts (Fα, F, Fβ) im Augengewebe (6) ausrichtet.

9. Ophthalmologische Vorrichtung (1) nach einem der Ansprüche 5 oder 6, **gekennzeichnet durch** ein Steuermodul (23), das eingerichtet ist, die Linearantriebe (21, 22) derart zu steuern, dass die Linearantriebe (21, 22) den Spiegel (20) parallel an einen Schnittpunkt (A-, A, A+) mit der optischen Achse (v) der Projektionsoptik (12) verschieben, welcher eine definierte Distanz (d-, d, d+) zur Brennweite (f) der Projektionsoptik (12) aufweist, und den Spiegel (20) so um den Schnittpunkt (A-, A, A+) dreht, dass die Laserpulse in Richtung eines die definierte Distanz (d-, d, d+) zum Schnittpunkt (A-, A, A+) aufweisenden Zielpunkts (Fα, F, Fβ) im Augengewebe (6) abgelenkt werden.

10. Ophthalmologische Vorrichtung (1) nach einem der Ansprüche 7 bis 9, **dadurch gekennzeichnet, dass** das Steuermodul (23) eingerichtet ist, das Antriebssystem (200) und die Scannvorrichtung (2) derart zu steuern, dass die Laserpulse auf Zielpunkte (Fα, F, Fβ) einer dreidimensionalen Bearbeitungsfläche im Augengewebe (6) abgelenkt werden.

11. Ophthalmologische Vorrichtung (1) nach einem der Ansprüche 1 bis 10, **gekennzeichnet durch** ein Korrektursystem, das eingerichtet ist, mittels eines der Projektionsoptik (12) vorgeschalteten optischen Elements, eine von der Ablenkung der Laserpulse abhängige Brennweitenänderung vorzunehmen.

12. Ophthalmologische Vorrichtung (1) nach Anspruch 11, **dadurch gekennzeichnet, dass** das Korrektursystem einen der Projektionsoptik (12) vorgeschalteten Divergenzmodulator (81) umfasst, welcher eingerichtet ist, die Divergenz des Laserstrahls abhängig von der Ablenkung der Laserpulse zu verändern.

13. Ophthalmologische Vorrichtung (1) nach Anspruch 12, **dadurch gekennzeichnet, dass** der Divergenzmodulator (81) mindestens eines aus der folgenden Liste umfasst: zwei seriell angeordnete optische Linsen, wobei mindestens eine der Linsen zur Modulation der Divergenz des Laserstrahls, auf einer optischen Achse (v) verschiebbar, mit einem Bewegungstreiber gekoppelt ist, eine verformbare Linse, ein verformbares Spiegelelement, ein räumlicher Lichtmodulator zum Modulieren der Wellenfront des Laserstrahls, ein Flächenlichtmodulator zum Modulieren der Reflexionswinkel an mehreren Punkten einer Reflexionsfläche, ein Brechungsmodulator zum Modulieren des Brechungsindexes eines optischen Elements an mehreren Punkten im Querschnitt des Strahlengangs, und ein Amplitudenmodulator zur Amplitudenmodulation an mehreren Punkten im Querschnitt des Strahlengangs des Laserstrahls.

14. Ophthalmologische Vorrichtung (1) nach einem der Ansprüche 1 bis 13, **gekennzeichnet durch** ein der Projektionsoptik (12) vorgeschaltetes oder in die Projektionsoptik (12) integriertes Zoomsystem (83), das eingerichtet ist, eine von der Ablenkung der Laserpulse abhängige Brennweitenänderung vorzunehmen.

15. Ophthalmologische Vorrichtung (1) nach einem der Ansprüche 1 bis 14, **gekennzeichnet durch** ein Kompensationssystem (7), das bewegbare Massen (70) zur Kompensation von durch bewegte optische Elemente verursachten Beschleunigungskräften umfasst.

## Claims

1. Ophthalmic device (1) for treating eye tissue (6) using laser pulses, comprising:
a projection optical unit (12) for focussed projection of the laser pulses into the eye tissue and
a scanning device (2), with a movable mirror (20), arranged downstream from the projection optical unit (12), for deflecting the laser pulses projected by the projection optical unit (12) in at least one deflection direction by rotating the mirror (20) around at least one axis of rotation,
**characterized by**
a drive system (200) configured to displace the mirror (20) in parallel.

2. Ophthalmic device (1) according to Claim 1, **characterized in that** the drive system (200) comprises a drive (24) coupled to the scanning device (2), which drive is configured to displace the scanning device (2) along the optical axis (v) of the projection optical unit (12) or transversely to the optical axis (v) of the projection optical unit (12).

3. Ophthalmic device (1) according to Claim 1, **characterized in that** the drive system (200) comprises at least one drive (21, 22) coupled to the mirror (20), which drive is configured to displace the mirror (20) in parallel.

4. Ophthalmic device (1) according to one of Claims 1 to 3, **characterized in that** the mirror (20) has a pivot point (A) lying on the optical axis (v) of the projection optical unit (12) and **in that** the drive system (200) is configured to displace the mirror (20) and the pivot point (A) along the optical axis (v) of the projection optical unit (12) .

5. Ophthalmic device (1) according to Claim 1, **characterized in that** the drive system (200) comprises a plurality of linear drives (21, 22) coupled to the mirror (20), which drives are configured to displace the mirror (20) in parallel and to rotate the mirror (20) around at least one pivot point (A, B).

6. Ophthalmic device (1) according to Claim 1, **characterized in that** the drive system (200) comprises a plurality of linear drives (21, 22) coupled to the mirror (20), which drives are configured to displace the mirror (20) in parallel and to rotate the mirror (20) around a pivot point (A) lying on the optical axis (v) of the projection optical unit (12).

7. Ophthalmic device (1) according to one of Claims 1 to 6, **characterized by** a control module (23) configured to control the drive system (200) in such a way that the drive system (200) displaces the mirror (20) in parallel to a point of intersection (A-, A, A+) with the optical axis (v) of the projection optical unit (12), which point of intersection has a defined distance (d-, d, d+) with respect to the focal length (f) of the projection optical unit (12).

8. Ophthalmic device (1) according to one of Claims 1 to 6, **characterized by** a control module (23) configured to control the drive system (200) in such a way that the drive system (200) displaces the mirror (20) in parallel to a point of intersection (A-, A, A+) with the optical axis (v) of the projection optical unit (12), which point of intersection has a defined distance (d-, d, d+) with respect to the focal length (f) of the projection optical unit (12), and to control the scanning device (2) in such a way that the scanning device (2) aligns the mirror (20) for deflecting the laser pulses in the direction of a target point (Fα, F, Fβ) in the eye tissue (6), which target point has the defined distance (d-, d, d+) from the point of intersection (A-, A, A+).

9. Ophthalmic device (1) according to either Claim 5 or 6, **characterized by** a control module (23) configured to control the linear drives (21, 22) in such a way that the linear drives (21, 22) displace the mirror (20) in parallel to a point of intersection (A-, A, A+) with the optical axis (v) of the projection optical unit (12), which point of intersection has a defined distance (d-, d, d+) with respect to the focal length (f) of the projection optical unit (12), and rotates the mirror (20) around the point of intersection (A-, A, A+) in such a way that the laser pulses are deflected in the direction of a target point (Fα, F, Fβ) in the eye tissue (6), which target point has the defined distance (d-, d, d+) from the point of intersection (A-, A, A+).

10. Ophthalmic device (1) according to one of Claims 7 to 9, **characterized in that** the control module (23) is configured to control the drive system (200) and the scanning device (2) in such a way that the laser pulses are deflected onto target points (Fα, F, Fβ) of a three-dimensional treatment surface in the eye tissue (6).

11. Ophthalmic device (1) according to one of Claims 1 to 10, **characterized by** a correction system configured to undertake a change in focal length depending on the deflection of the laser pulses by means of an optical element arranged upstream from the projection optical unit (12).

12. Ophthalmic device (1) according to Claim 11, **characterized in that** the correction system comprises a divergence modulator (81) arranged upstream from the projection optical unit (12), which divergence modulator is configured to modify the divergence of the laser beam depending on the deflection of the laser pulses.

13. Ophthalmic device (1) according to Claim 12, **characterized in that** the divergence modulator (81) comprises at least one item from the following list: two optical lenses arranged in series, wherein at least one of the lenses is coupled to a movement driver in a manner displaceable on an optical axis (v) for modulating the divergence of the laser beam, a deformable lens, a deformable mirror element, a spatial light modulator for modulating the wavefront of the laser beam, an area light modulator for modulating the reflection angles at a plurality of points of a reflection surface, a refraction modulator for modulating the refractive index of an optical element at a plurality of points in the cross section of the beam path, and an amplitude modulator for modulating the amplitude at a plurality of points in the cross section of the beam path of the laser beam.

14. Ophthalmic device (1) according to one of Claims 1 to 13, **characterized by** a zoom system (83) arranged upstream from the projection optical unit (12) or integrated into the projection optical unit (12), which zoom system is configured to undertake a change in focal length depending on the deflection of the laser pulses.

15. Ophthalmic device (1) according to one of Claims 1 to 14, **characterized by** a compensation system (7) comprising movable masses (70) for compensating for acceleration forces caused by moved optical elements.

## Revendications

1. Dispositif ophtalmologique (1) pour le traitement de tissus oculaires (6) au moyen d'impulsions laser, comprenant :
une optique de projection (12) pour la projection focalisée des impulsions laser dans le tissu oculaire, et
un dispositif de balayage (2) monté en aval de l'optique de projection (12) et comportant un miroir mobile (20) pour la déviation des impulsions laser projetées par l'optique de projection (12) dans au moins une direction de déviation par rotation du miroir (20) autour d'au moins un axe de rotation,
**caractérisé par** un système d'entraînement (200) qui est conçu pour déplacer le miroir (20) parallèlement.

2. Dispositif ophtalmologique (1) selon la revendication 1, **caractérisé en ce que** le système d'entraînement (200) comprend un entraînement (24) couplé au dispositif de balayage (2), qui est conçu pour déplacer le dispositif de balayage (2) le long de l'axe optique (v) de l'optique de projection (12) ou transversalement à l'axe optique (v) de l'optique de projection (12).

3. Dispositif ophtalmologique (1) selon la revendication 1, **caractérisé en ce que** le système d'entraînement (200) comprend au moins un entraînement (21, 22) couplé au miroir (20), qui est conçu pour déplacer le miroir (20) parallèlement.

4. Dispositif ophtalmologique (1) selon l'une des revendications 1 à 3, **caractérisé en ce que** le miroir (20) présente un point de rotation (A) situé sur l'axe optique (v) de l'optique de projection (12), et **en ce que** le système d'entraînement (200) est conçu pour déplacer le miroir (20) et le point de rotation (A) le long de l'axe optique (v) de l'optique de projection (12).

5. Dispositif ophtalmologique (1) selon la revendication 1, **caractérisé en ce que** le système d'entraînement (200) comprend une pluralité d'entraînements linéaires (21, 22) couplés au miroir (20), qui sont conçus pour déplacer le miroir (20) parallèlement et pour faire tourner le miroir (20) autour d'au moins un point de rotation (A, B).

6. Dispositif ophtalmologique (1) selon la revendication 1, **caractérisé en ce que** le système d'entraînement (200) comprend une pluralité d'entraînements linéaires (21, 22) couplés au miroir (20), qui sont conçus pour déplacer le miroir (20) parallèlement et pour faire tourner le miroir (20) autour d'un point de rotation (A) situé sur l'axe optique (v) de l'optique de projection (12).

7. Dispositif ophtalmologique (1) selon l'une des revendications 1 à 6, **caractérisé par** un module de commande (23) qui est conçu pour commander le système d'entraînement (200) de manière à ce que le système d'entraînement (200) déplace le miroir (20) parallèlement, en un point d'intersection (A-, A, A+) avec l'axe optique (v) de l'optique de projection (12) qui présente une distance définie (d-, d, d+) par rapport à la longueur focale (f) de l'optique de projection (12).

8. Dispositif ophtalmologique (1) selon l'une des revendications 1 à 6, **caractérisé par** un module de commande (23) qui est conçu pour commander le système d'entraînement (200) de manière à ce que le système d'entraînement (200) déplace le miroir (20) parallèlement, en un point d'intersection (A-, A, A+) avec l'axe optique (v) de l'optique de projection (12) qui présente une distance définie (d-, d, d+) par rapport à la longueur focale (f) de l'optique de projection (12), et pour commander le dispositif de balayage (2) de manière à ce que le dispositif de balayage (2) oriente le miroir (20) afin de dévier les impulsions laser dans la direction d'un point cible (Fα, F, Fβ) dans le tissu oculaire (6) présentant la distance définie (d-, d, d+) par rapport au point d'intersection (A-, A, A+).

9. Dispositif ophtalmologique (1) selon l'une des revendications 5 ou 6, **caractérisé par** un module de commande (23) qui est conçu pour commander les entraînements linéaires (21, 22) de manière à ce que les entraînements linéaires (21, 22) déplacent le miroir (20) parallèlement, en un point d'intersection (A-, A, A+) avec l'axe optique (v) de l'optique de projection (12) qui présente une distance définie (d-, d, d+) par rapport à la longueur focale (f) de l'optique de projection (12), et fait tourner le miroir (20) autour du point d'intersection (A-, A, A+) de manière à ce que les impulsions laser soient déviées vers un point cible (Fα, F, Fβ) dans le tissu oculaire (6) présentant la distance définie (d-, d, d+) par rapport au point d'intersection (A-, A, A+).

10. Dispositif ophtalmologique (1) selon l'une des revendications 7 à 9, **caractérisé en ce que** le module de commande (23) est conçu pour commander le système d'entraînement (200) et le dispositif de balayage (2) de manière à ce que les impulsions laser soient déviées dans la direction de points cibles (Fα, F, Fβ) d'une surface de traitement tridimensionnelle dans le tissu oculaire (6) .

11. Dispositif ophtalmologique (1) selon l'une des revendications 1 à 10, **caractérisé par** un système de correction qui est conçu pour effectuer une variation de focale en fonction de la déviation des impulsions laser au moyen d'un élément optique monté en amont de l'optique de projection (12).

12. Dispositif ophtalmologique (1) selon la revendication 11, **caractérisé en ce que** le système de correction comprend un modulateur de divergence (81) monté en amont de l'optique de projection (12), qui est conçu pour modifier la divergence du faisceau laser en fonction de la déviation des impulsions du laser.

13. Dispositif ophtalmologique (1) selon la revendication 12, **caractérisé en ce que** le modulateur de divergence (81) comprend au moins l'un des éléments de la liste suivante : deux lentilles optiques disposées en série, dans lequel au moins l'une des lentilles est couplée à un dispositif pilote de mouvement pour moduler la divergence du faisceau laser et peut être déplacée sur un axe optique (v), une lentille déformable, un élément miroir déformable, un modulateur spatial de lumière pour la modulation du front d'onde du faisceau laser, un modulateur zonal de lumière pour la modulation des angles de réflexion en plusieurs points d'une surface de réflexion, un modulateur de réfraction pour la modulation de l'indice de réfraction d'un élément optique en une pluralité de points dans la section transversale du chemin de faisceau, et un modulateur d'amplitude pour la modulation d'amplitude en une pluralité de points dans la section transversale du chemin de faisceau du faisceau laser.

14. Dispositif ophtalmologique (1) selon l'une des revendications 1 à 13, **caractérisé par** un système à focale variable (83) qui est monté en amont de l'optique de projection (12) ou intégré dans l'optique de projection (12), qui est conçu pour effectuer une variation de focale en fonction de la déviation des impulsions du laser.

15. Dispositif ophtalmique (1) selon l'une quelconque des revendications 1 à 14, **caractérisé par** un système de compensation (7) qui comprend des masses mobiles (70) pour la compensation de forces d'accélération provoquées par des éléments optiques mobiles.
